Europäisches Patentamt

European Patent Office

Office européen des brevets

(11) Veröffentlichungsnummer: **0 301 374 B1**

## (12) EUROPÄISCHE PATENTSCHRIFT

(45) Veröffentlichungstag der Patentschrift: **03.02.93**

(21) Anmeldenummer: **88111576.0**

(22) Anmeldetag: **19.07.88**

(51) Int. Cl.⁵: **C07K 3/18**, //A61K35/50

54 **Verfahren zur Reinigung des plazentaren Gewebeproteins PP4.**

(30) Priorität: **25.07.87 DE 3724726**

(43) Veröffentlichungstag der Anmeldung:
**01.02.89 Patentblatt 89/05**

(45) Bekanntmachung des Hinweises auf die
Patenterteilung:
**03.02.93 Patentblatt 93/05**

(84) Benannte Vertragsstaaten:
**AT BE CH DE ES FR GB GR IT LI LU NL SE**

(56) Entgegenhaltungen:
**EP-A- 0 123 307**
**EP-A- 0 203 463**

**ARCHIVES OF GYNECOLOGY, Band 236,
1985, Seiten 225-233, Springer-Velag; H.
BOHN et al.: "Isolation and characterization
of two membrane-associated placental tissue proteins"**

(73) Patentinhaber: **BEHRINGWERKE Aktiengesellschaft
Postfach 1140
W-3550 Marburg 1(DE)**

(72) Erfinder: **Löbermann, Hartmut, Dr.
Mooswaldstrasse 7B
W-7801 Schallstadt(DE)**

(74) Vertreter: **Fischer, Hans-Jürgen, Dr. et al
Hoechst AG Zentrale Patentabteilung Postfach 80 03 20
W-6230 Frankturt am Main 80(DE)**

**Beschreibung**

Die Erfindung betrifft ein Verfahren zur Reinigung des plazentaren Gewebeproteins PP4, das gerinnungshemmende Eigenschaften besitzt.

Das Gewebeprotein PP4 ist in DE 33 15 000 A 1 (USP 4,507,229) mit folgenden Parametern beschrieben:
- einer elektrophoretischen Beweglichkeit im Bereich zwischen der von $alpha_1$ und $alpha_2$ Globulinen;
- einem isoelektrischen Punkt von $4,85 \pm 0,15$;
- einem Sedimentationskoeffizienten

$$s^0_{20,w}$$

von $3,3 \pm 0,2S$;
- einem im natriumdodecylsulfat (SDS)-haltigen Polyacrylamidgel bestimmtes Molekulargewicht von $35.000 \pm 5.000$;
- einem Extinktionskoeffizienten

$$E^{1\%}_{1cm} (280 \text{ nm})$$

von $5,9 \pm 0,6$;
- einem Kohlenhydratanteil von $2,4 \pm 0,94$ % (g/100 g) (Mannose $0,3 \pm 0,2$ %, Galactose $0,4 \pm 0,2$ %, Xylose $0,1 \pm 0,04$ %, Glukose $0,2 \pm 0,1$ %, Glukosamin $1,0 \pm 0,2$ %, Neuraminsäure $0,4 \pm 0,2$ %) und
- der folgenden Aminosäurenzusammensetzung:

| Aminosäure | Reste pro 100 Reste(Mol %) | Variationskoeffizient |
|---|---|---|
| Lysin | 6,95 | 1,14 |
| Histidin | 0,97 | 17,40 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Glycin | 6,68 | 3,83 |
| Alanin | 7,92 | 1,67 |
| Cystin 1/2 | 0,77 | 19,50 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,90 |

Die bisherigen Verfahren zur Reinigung von PP4, wie beispielsweise Immunadsorption, Gelfiltration etc. (Bohn et al. 1985, Arch. Gynaecol. 236, 225-233) sind für eine großtechnische Isolierung von PP4 nicht geeignet.

Aufgabe der Erfindung war daher die Entwicklung eines Verfahrens zur Reinigung von PP4.

Es wurde nun überraschenderweise gefunden, daß PP4 in Gegenwart von Calciumionen Affinität zu Polyschwefelsäureestern von Sacchariden oder zu sulfatierten Zuckern aufweist und an trägergebundene Polyschwefelsäureester von Sacchariden oder sulfatierte Zucker gebunden werden kann.

Gegenstand der Erfindung ist somit ein Verfahren zur Reinigung von PP4, dadurch gekennzeichnet, daß eine PP4-haltige Lösung in Gegenwart von Calciumionen mit einem trägergebundenen Polyschwefelsäureester eines Saccharides oder sulfatierten Zucker in Kontakt gebracht, die überstehende Flüssigkeit abgetrennt, die mit PP4 beladene Trägermatrix gegebenenfalls gewaschen und PP4 eluiert wird.

Als wasserunlösliche Trägermatrix, an die ein Polyschwefelsäureester eines Saccharides oder ein sulfatierter Zucker kovalent gebunden werden können, werden beispielsweise unlösliche Agarose, Dextran, Polyacrylamid, ein aminofunktionalisiertes Trägerharz oder Copolymere des Polyethylenglykols, Pentaerythrits und Methacrylats oder auch deren Kombinationen verwendet.

Die Kupplung eines Polyschwefelsäureesters eines Saccharides oder sulfatierten Zuckers an einen solchen Träger kann nach bekannten Methoden, beispielsweise durch Reaktion mit einem mit CNBr oder Epoxid voraktivierten Trägerharz oder über Carbodiimid-Kupplung an aminofunktionalisiertes Trägerharz durchgeführt werden.

In einer bevorzugten Verfahrensweise wird eine PP4-haltige Lösung von pH 5,5 - 9,5, die vorzugsweise 0,01 - 50 mg PP4/ml Lösung und 0,001 - 0,2 mol/l Calciumionen, beispielsweise in Form von Calciumchlorid, Calciumacetat oder Calciumlactat enthält, mit einem trägergebundenen Polyschwefelsäureester eines Saccharides oder trägergebundenem sulfatierten Zucker, beispielsweise trägergebundenem Heparin, Dextransulfat, Heparansulfat, Chondroitinsulfat, Keratansulfat oder Dermatansulfat, in Kontakt gebracht, die überstehende Lösung abgetrennt, das mit PP4 beladene Trägermaterial gegebenenfalls mit einem Puffer pH 5,5 - 9,5, der 0,001 - 0,2 mol/l Calciumionen und 0,001 - 0,4 mol/l eines Salzes beispielsweise NaCl, KCl oder LiCl enthält, gewaschen und PP4 durch eine Stufen- oder Gradientenelution unter Erhöhung der Salzkonzentration eluiert.

In einer besonders bevorzugten Verfahrensweise wird eine PP4-enthaltende Lösung von pH 7,0 - 9,0, die 0,003 - 0,02 mol/l Calciumionen enthält, mit trägergebundenem Heparin oder Dextransulfat, ganz besonders bevorzugt aber mit trägergebundenem Heparin in Kontakt gebracht, die Mischung beispielsweise 5 - 300 min bei 4 - 30°C inkubiert, die überstehende Lösung von dem mit PP4 beladenen Trägerharz abgetrennt, gegebenenfalls mit einer Lösung von pH 7,0 - 9,0, die 0,003 - 0,02 mol/l Calciumionen und 0,05 - 0,3 mol/l NaCl, KCl oder LiCl enthält, gewaschen und PP4 durch Erhöhung der Salzkonzentration auf 0,35 - 1,5 mol/l mittels einer Stufen- oder Gradientenelution vom Trägerharz abgelöst.

Es kann gegebenenfalls beispielsweise auch eine Rechromatographie an einem anderen trägergebundenen Polyschwefelsäureester eines Saccharides oder trägergebundenen sulfatierten Zucker angeschlossen werden.

Das erfindungsgemäße Verfahren zeichnet sich dadurch aus, daß sich PP4 in wenigen Schritten in sehr hoher Reinheit und Ausbeute auch im großtechnischen Maßstab isolieren läßt.

Die Erfindung soll durch die folgenden Beispiele erläutert werden.

Beispiel 1

3 l eines Phenyl-[R]Sepharose-Eluates, das gemäß Beispiel 1 der DE-A-35 43 182 hergestellt worden waren (160 µg PP4/ml), wurden gegen Puffer A dialysiert (50 mmol/l Tris/HCl, 25 mmol/l NaCl, 5 mmol/l CaCl$_2$, pH 8,0). Die Lösung wurde im Batch-Verfahren mit 300 ml einer Heparin-[R]Sepharose (Fa. Pharmacia, Uppsala, Schweden, Best.Nr. 17-0467-01) versetzt, 30 min bei Raumtemperatur gerührt und die überstehende Lösung abgetrennt. Das Adsorbens wurde danach mit Puffer A gewaschen und PP4 durch einen NaCl-Stufengradienten (180 mmol/l, 3000 mmol/l und 1,5 mol/l NaCl) eluiert. Gegebenenfalls kann zur weiteren Reinigung das so erhaltene PP4-Material an einer Dextransulfat-[R]Sepharose in Puffer A ohne CaCl$_2$ rechromatographiert werden. Das auf diese Weise gereinigte PP4 ergab in der SDS-Polyacrylamid-Gelelektrophorese eine Bande mit einem Molekulargewicht von ca. 32 kDa.

Beispiel 2

1 l Phenyl-[R]SepharoseEluat (wie Beispiel 1) wurde gegen Puffer A dialysiert. Das Dialysat wurde auf 200 ml Dextransulfat-[R]Sepharose (Epoxy-aktivierte Sepharose 6B, Dextransulfat von Fa. Pharmacia, Upsala, Schweden, Best.Nr. 17-0480-01, 17-0340-01; Kupplung der Substanzen erfolgte nach bekannten Methoden, beispielsweise nach Sundberg, L., Porath` J. (1974) J.Chromatogr. 90, 87-98.), die mit Puffer A äquilibriert war, aufgetragen und gewaschen. PP4 wurde mittels eines NaCl-Gradienten eluiert. Die PP4-enthaltenden Fraktionen wurden nach Dialyse gegen Puffer A beispielsweise an einer Heparin-[R]Sepharose rechromatographiert. Das PP4-Material ergab in einer SDS-Polyacrylamid-Gelelektrophorese eine Bande mit einem Molekulargewicht von ca. 32 kDa.

**Patentansprüche**

1. Verfahren zur Reinigung von PP4, dadurch gekennzeichnet, daß eine PP4-haltige Lösung in Gegenwart von Calciumionen mit einem trägergebundenen Polyschwefelsäureester eines Saccharides oder sulfatierten Zucker in Kontakt gebracht, die überstehende Flüssigkeit abgetrennt, die mit PP4 beladene Trägermatrix gegebenenfalls gewaschen und das PP4 eluiert wird.

2. Verfahren nach Anspruch 1, dadurch gekennzeicnet, daß das Trägermaterial unlösliche Agarose, Dextran, Polyacrylamid, ein aminofunktionalisiertes Trägerharz oder ein Copolymeres des Polyethylenglykols, Pentaerythrits und Methacrylats oder eine Kombination von diesen ist.

3. Verfahren nach Anspruch 1, dadurch gekennzeichnet, daß der Polyschwefelsäureester eines Saccharides Heparin, Dextransulfat, Heparansulfat, Chondroitinsulfat, Keratansulfat oder Dermatansulfat ist.

4. Verfahren nach Anspruch 1, dadurch gekennzeichnet, daß trägergebundenes Heparin oder Dextransulfat, vorzugsweise trägergebundenes Heparin verwendet wird.

5. Verfahren nach Anspruch 1, dadurch gekennzeichnet, daß 0,001 - 0,2 mol/l Calciumionen anwesend sind.

6. Verfahren nach Anspruch 1, dadurch gekennzeichnet, daß trägergebundenes Heparin, Dextransulfat, Heparansulfat, Chondroitinsulfat, Keratansulfat oder Dermatansulfat verwendet wird.

7. Verfahren nach Anspruch 1, dadurch gekennzeichnet, daß mit einem Puffer von pH 5,5 - 9,5, der 0,001 - 0,2 mol/l, vorzugsweise 0,003 - 0,02 mol/l Calciumionen und 0,001 - 0,4 mol/l, vorzugsweise 0,05 - 0,3 mol/l NaCl, KCl oder LiCl enthält, gewaschen wird.

## Claims

1. A process for the purification of PP4, which comprises contacting a PP4-containing solution, in the presence of calcium ions, with a carrier-bound saccharide polysulfate or sulfated sugar, removing the supernatant liquid, where appropriate washing the carrier matrix loaded with PP4, and eluting PP4.

2. The process as claimed in claim 1, wherein the carrier material is insoluble agarose, dextran, polyacrylamide, an amino-functionalized carrier resin or a copolymer of polyethylene glycol, pentaerythritol and methacrylate, or a combination of these.

3. The process as claimed in claim 1, wherein the saccharide polysulfate is heparin, dextran sulfate, heparan sulfate, chondroitin sulfate, keratan sulfate or dermatan sulfate.

4. The process as claimed in claim 1, wherein carrier-bound heparin or dextran sulfate, preferably carrier-bound heparin, is used.

5. The process as claimed in claim 1, wherein 0.001 - 0.2 mol/l calcium ions are present.

6. The process as claimed in claim 1, wherein carrier-bound heparin, dextran sulfate, heparan sulfate, chondroitin sulfate, keratan sulfate or dermatan sulfate is used.

7. The process as claimed in claim 1, wherein a buffer of pH 5.5 - 9.5, which contains 0.001 - 0.2 mol/l, preferably 0.003 - 0.02 mol/l, calcium ions and 0.001 - 0.4 mol/l, preferably 0.05 - 0.3 mol/l, NaCl, KCl or LiCl, is used for washing.

## Revendications

1. Procédé de purification de PP4, caractérisé en ce qu'on met une solution contenant PP4, en présence d'ions calcium, en contact avec un ester de l'acide polysulfurique et d'un saccharide ou avec un sucre sulfaté, fixé à un substrat, on sépare le liquide surnageant, on lave éventuellement la matrice support chargée de PP4 et on élue PP4.

2. Procédé selon la revendication 1, caractérise en ce que le matériau support est de l'agarose insoluble,

4

du dextran, du polyacrylamide, une résine support à fonction amino, ou un copolymère de poly-(éthylèneglycol), de pentaérythritol et de méthacrylate, ou une de leurs combinaisons.

3. Procédé selon la revendication 1, caractérisé en ce que l'ester de l'acide polysulfurique et d'un saccharide est de l'héparine, du sulfate de dextran, du sulfate d'héparan, du sulfate de chondroïtine, du sulfate de kératan ou du sulfate de dermatan.

4. Procédé selon la revendication 1, caractérisé en ce qu'on utilise l'héparine ou le sulfate de dextran fixé-(e) à un support, de préférence l'héparine fixée à un support.

5. Procédé selon la revendication 1, caractérisé en ce les ions calcium sont présents a raison de 0,001 a 0,2 mole/l.

6. Procédé selon la revendication 1, caractérisé en ce qu'on utilise de l'héparine, du sulfate de dextran, du sulfate d'héparan, du sulfate de chondroïtine, du sulfate de kératan ou du sulfate de dermatan, fixé(e) à un support.

7. Procédé selon la revendication 1, caractérisé en ce qu'on effectue le lavage avec un tampon pH 5,5-9,5 qui contient de 0,001 a 0,2 mole/l, de préférence de 0,003 à 0,02 mole/l d'ions calcium, et de 0,001 a 0,4 mole/l, de préférence de 0,05 à 0,3 mole/l de NaCl, KCl ou LiCl.